# EUROPEAN PATENT APPLICATION

(11) **EP 2 221 379 A1**
(43) Date of publication of application: **25.08.2010**
(21) Application number: 08848929.9
(22) Date of filing: 13.11.2008
(51) Int. Cl.: C12N 15/12, C12N 15/85, C07K 14/47, C12Q 1/66

(54) **METHOD OF IDENTIFYING COMPOUNDS THAT INDUCE OR INHIBIT ENDOPLASMIC RETICULUM STRESS OR OXIDATIVE STRESS**

(30) Priority: 13.11.2007 ES 200702997
(71) Applicant: Universidad Autónoma de Madrid, 28049 Madrid (ES)
(72) Inventor: VALDIVIESO ÁMATE, Fernando, E-28049 Madrid (ES); BULLIDO GÓMEZ-HERAS, María Jesús, E-28049 Madrid (ES); MARTÍNEZ GARCÍA, Ana, E-28049 Madrid (ES)
(74) Representative: ABG Patentes, S.L.
(86) International application number: PCT/ES2008/000705
(87) International publication number: WO 2009/063110

(57) **Abstract**

The present invention generally relates to a method for the identification of compounds for the treatment of neurodegenerative diseases. Said method is based on a cell model for neurodegeneration useful for the identification of potentially useful compounds for the treatment or the prevention of a neurodegenerative disease associated with deficient expression of the *DDIT3* gene, also known as *CHOP.*

## Description

The present invention generally relates to a method for the identification of compounds for the treatment of neurodegenerative diseases. Said method is based on a cell model for neurodegeneration useful for the identification of potentially useful compounds for the treatment or the prevention of a neurodegenerative disease.

### Background of the Invention

Neurodegenerative diseases are chronic and progressive processes which are characterized by selective and symmetrical losses of neurons in the motor, sensory and cognitive systems. These diseases are growing at an alarming rate in developed countries due to several factors, including the gradual aging of the population and changes in lifestyle. There is still no suitable treatment for said diseases.

### Oxidative stress (OS)

Reactive oxygen species (ROS), such as the oxygen radical, superoxide (O²⁻), or hydrogen peroxide (H₂O₂), are produced during normal metabolic processes and they perform several functions useful. Cells are provided with several mechanisms for controlling the levels of these oxidative agents, for example, superoxide dismutase (SOD), glutathione or vitamin E. In normal physiological conditions, there is a balance between ROS and these antioxidative mechanisms. Excessive production of ROS and a loss of efficacy of the antioxidative defenses can lead to oxidative cellular stress and therefore, to pathological states in cells and cause tissue damage. This event seems to occur in a more spectacular manner in neurons due to their high rate of metabolic activity and, therefore, seems to be related to a series of degenerative processes, diseases and syndromes, for example, Alzheimer's disease (AD), Parkinson's disease (PD), amyotrophic lateral sclerosis (ALS) and schizophrenia. Treatments leading to an enhancement of the antioxidative mechanisms can slow the progression of some of the mentioned diseases.

It is known that during normal aging the brain undergoes morphological and functional alterations affecting the dendritic trees, synapses, neurotransmission, circulation and metabolism, and these changes are reflected in the motor and sensory systems, in sleep, memory and learning. The neuronal changes occurring due to age include the reduction of Ca²⁺ homeostasis and of the sensitivity of the dopaminergic, cholinergic and opioid systems and of catecholamines. With increasing frequency, it seems that greater susceptibility to the long-term effects of OS, mitochondrial dysfunction (which increases with age) and damage due to inflammation is involved in these alterations.

### Endoplasmic Reticulum Stress (ERS)

The endoplasmic reticulum (ER) is where protein synthesis and post-translational changes for correct protein folding take place, and it is the common transport pathway for supplying proteins to their suitable destination inside the cell, and it is also a Ca²⁺ deposit. The high concentration of Ca²⁺ existing in the lumen of the ER with respect to the concentration in the cell cytoplasm is important both for cell signaling and for the correct processing of newly synthesized proteins. The ER performs quality control on the proteins, assuring correct processing of the end product, which is crucial for the normal operation of the cell. Only those proteins which, due to post-translational modifications, are correctly folded pass on to the Golgi apparatus, whereas those proteins which have not completed their folding or are incorrectly folded are retained in the ER to complete the process or they are marked for their degradation. In addition to this protein folding function, the ER is where sterols and lipids are synthesized. Any alteration in any of these processes is a cause of ERS.

Various physiological or pathological situations affecting protein folding and/or Ca²⁺ homeostasis, such as glucose deprivation, viral infections or the expression of mutant proteins unable to fold, can be a cause of ERS due to the accumulation of poorly folded proteins, and then a response to this stress occurs in the cells, the unfolded protein response (UPR) being activated. Protein folding *in vivo* requires folding machinery in the ER which can be overloaded, resulting in an accumulation of poorly folded proteins, and then the UPR response is activated to attempt to re-establish the normal state in the ER.

In these cases, the folding capacity of the ER can be increased increasing the expression of its folding machinery and of chaperones such as BiP/GRP78 and GRP94 and increasing the size of the ER and, on the other hand, decreasing the protein load by means of translation and transcription silencing and eliminating the proteins that are poorly folded and marked as such. When these mechanisms are unable to remedy the stress situation, the ER initiates and alarm signal to the immune system through NF-κB, and excessive and/or prolonged ERS leads to "cell suicide", usually due to an apoptotic process.

A common sign of neurodegenerative diseases is the accumulation and deposits of poorly folded proteins affecting several signaling pathways which ultimately lead to neuronal death. Some authors consider that ER stress is related to several neurodegenerative diseases such as AD, PD, ALS, Huntington's and transmissible spongiform encephalopathies (TSE).

Therefore, there is a need to develop new processes for the identification of therapeutic compounds for the treatment of neurodegenerative diseases, particularly of neurodegenerative diseases presenting oxidative stress and/or endoplasmic reticulum stress and leading to neuronal death.

### Integrated Stress Response (ISR)

Eukaryotic cells respond to poorly folded proteins in the ER, amino acid deprivation or to oxidizing conditions by reversible phosphorylating factor eIF2α. This phosphorylation inhibits the general synthesis of proteins by promoting the expression of isoform 4 of the activating transcription factor (ATF4), and thus activating the expression of targets involved in UPR, such as Chop. It involves an integrated stress response (ISR) regulating the metabolism of amino acids and the resistance to oxidative stress, in addition to adapting cells to ERS. The cell can therefore steer towards apoptotic and/or autophagic processes in prolonged stress situations.

### DDIT3 (DNA-Damage-Inducible Transcript 3) gene

The *DDIT3,* (*DNA-Damage-Inducible Transcript* 3) gene, also known as *CHOP-10* (CCAAT/ *enhancer binding protein* (C/EBP) *Homologous Protein* -10), *CHOP, CEBPZ* (CEBPζ), *GADD153* (*Growth Arrest and DNA-Damage inducible gene* 153) or *MGC4154,* encodes for a protein commonly known as Chop.

Chop is a bZip transcription factor which is activated in response to agents which in some way alter the folding machinery of the ER. It can form homo- or heterodimers with other members of the C/EBP family, but the heterodimers does not bind to the classic C/EBP binding sites, such that, from the point of view of these target sites, Chop is an inhibitor of these transcription factors. However, the Chop homodimers are able to bind to other specific sequences different from the classic C/EBP sequences. Thus, Chop has a dual role, inhibiting the function of C/EBPs and activating other different target genes. It has been seen that the overexpression of CHOP and the microinjection of the Chop protein leads to cell cycle arrest and/or to apoptosis, whereas mice deficient in this gene show reduced apoptosis in response to ERS, and deficiencies in its expression may protect cells from the apoptosis caused by ERS.

An accumulation of poorly folded proteins is observed in neurodegenerative diseases. In the case of AD, it has been observed that mutations in the *PSEN1* gene increase the levels of Chop protein. In fact, it has been observed that an exaggerated response to stress occurs in cells with mutations in *PSEN1,* although such a high level of response was due to greater translation of the Chop messenger, more than to a higher level of transcription.

### Summary of the Invention

The present invention generally relates to a method for the identification of potentially useful compounds for the treatment or the prevention of a neurodegenerative disease comprising the steps of:
a) contacting a cell with a candidate compound in any degree of purity wherein said cell expresses a DNA construct comprising:
   i) the *DDIT3* gene promoter region identified in SEQ ID NO:
      1 or a functionally equivalent variant thereof; and
   ii) a reporter gene operatively coupled to said promoter region; and
b) detecting the expression of the reporter gene;
   wherein the assayed compound is identified as suitable for the treatment or prevention of said neurodegenerative disease if it causes a variation in the expression of the reporter gene in the direction opposite to the existing alteration of the levels of *DDIT3* in the neurodegenerative disease in comparison with the alteration of the expression in the absence of said compound, or if it causes an alteration in the expression of the reporter gene in the same direction as the alteration to be achieved in the levels of *DDIT3.*

In other additional aspects, the present invention relates to methods for the identification of compounds inducing or inhibiting endoplasmic reticulum stress and/or oxidative stress in a cell.

The present invention further relates to a method for the diagnosis of a neurodegenerative disease in a subject, or for determining the predisposition of a subject for suffering a neurodegenerative disease, or for determining the stage or severity of the disease in a subject, or for monitoring the effect of the therapy administered to a subject with said disease, comprising determining the presence or absence of the polymorphism identified in SEQ ID NO: 2 of *DDIT3* in a biological sample of said subject.

In another aspect, the present invention relates to a method for the regulated expression of a gene of interest comprising introducing in a cell a construct comprising the nucleotide sequence identified in SEQ ID NO: 1 or SEQ ID NO: 2.

In addition, the present invention relates to a gene construct comprising a DNA sequence comprising the nucleotide sequence identified in SEQ ID NO: 1 or a functionally equivalent variant thereof, as well as to an expression vector, to a cell and to a non-human animal comprising said construct.

### Brief Description of the Drawings

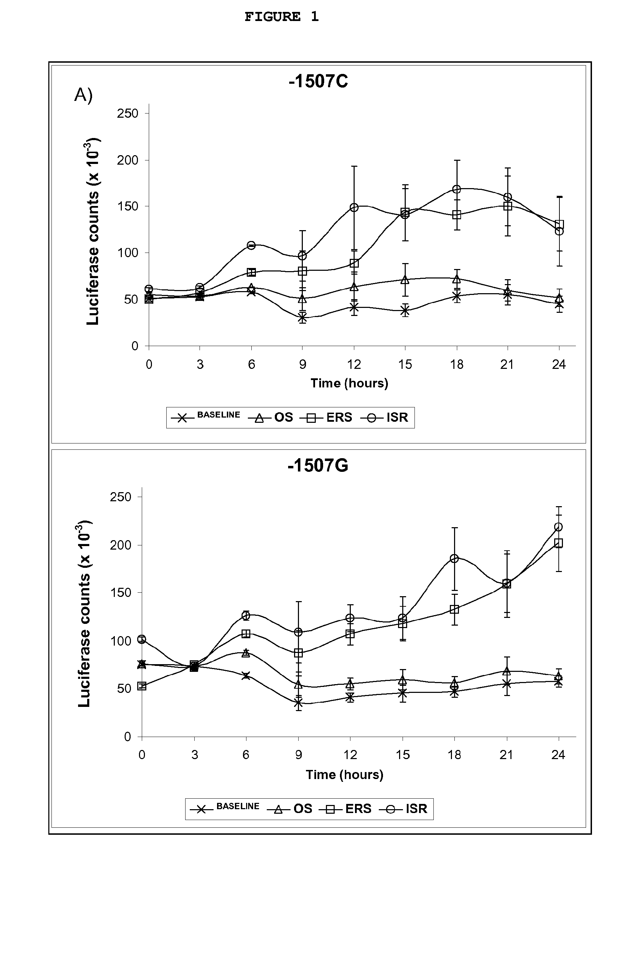

Figure 1 shows the transcriptional activity of the *DDIT3* promoter in SK-N-MC cells temporarily transfected with the two constructs pXP2 DDIT3(-1507C) - Luciferase (panel A) and *DDIT3*(-1507G) - Luciferase (panel B). It is shown in counts (x10⁻³) per mU of β-galactosidase in various culture conditions: baseline (cross), OS (triangle), ERS (square) and ISR (circle). The data shown are obtained from the average and standard errors of 7 kinetic experiments in triplicate.

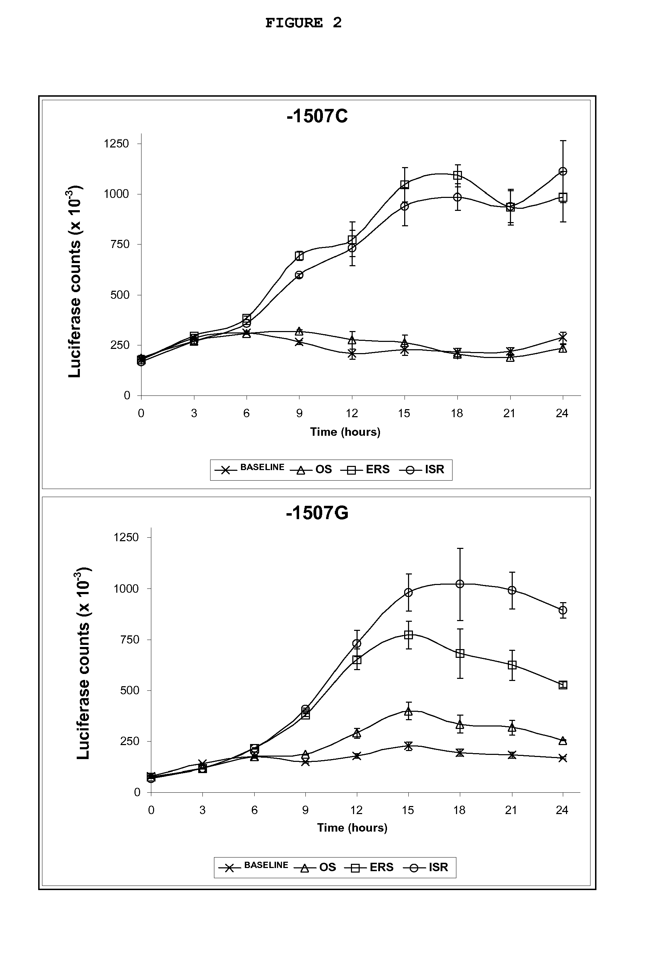

Figure 2 shows the transcriptional activity of the *DDIT3* promoter in SK-N-MC cells stably transfected with the two constructs pXP2 DDIT3(-1507C) - Luciferase (panel A) and *DDIT3*(-1507G) - Luciferase (panel B). It is shown in counts (x10⁻³) normalized by the number of cells in various culture conditions: baseline (cross), OS (triangle), ERS (square) and ISR (circle). The data shown are obtained from the mean and standard errors of 7 kinetic experiments in triplicate.

### Detailed Description of the Invention

The authors of the present invention have shown that when a cell line is stably transfected with a DNA construct comprising the nucleotide sequence of the *DDIT3* gene promoter identified in SEQ ID NO: 1 operatively coupled to a reporter gene, said cell line can be particularly useful for its use in screening methods for the identification of compounds activating or inhibiting the expression of the target gene, and therefore for the identification of potentially useful compounds for the treatment or the prevention of a neurodegenerative disease associated with a deficient expression of *DDIT3.*

As it is used herein, the term "neurodegenerative disease" relates to a disease associated with a progressive and specific loss of neurons (neuronal death), for example, Alexander disease, Alpers' disease, amyotrophic lateral sclerosis, ataxia-telangiectasia, Batten disease, spongiform encephalopathy (BSE), cocaine syndrome, corticobasal degeneration, Creutzfeldt-Jakob disease, Huntington's disease, HIV-associated dementia, Kennedy's disease, Krabbe disease, Lewy body dementia, Machado-Joseph disease, multiple sclerosis, multiple system atrophy, Neuroborreliosis, Parkinson's disease, Pelizaeus-Merzbacher disease, Pick's disease, primary lateral sclerosis, prion diseases, Refsum's disease, Sandhoff disease, Schilder's disease, schizophrenia, Spielmeyer-Vogt-Sjogren-Batten disease, spinocerebellar ataxia, spinal muscular atrophy, Steele-Richardson-Olszewski disease, Tabes dorsalis, inflammatory cerebral disease, etc.

The inventors have also surprisingly observed that a different capacity of response to stimuli occurs depending on the allelic variant of the *DDIT3* gene promoter present. Thus, the inventors have observed that in the event that said sequence presents the polymorphism identified in SEQ ID NO: 2 (variant - 1507G), a significant response to oxidative stress occurs, whereas said response to oxidative stress is not observed when the sequence of the promoter comprises the nucleotide sequence identified in SEQ ID NO: 1 (variant -1507C). In addition, the inventors have observed a greater response to endoplasmic reticulum stress in the event that the allelic variant of the promoter is the variant -1507C.

As it is used herein, the term "oxidative stress" (OS) relates to a cell alteration characterized by an excessive production of ROS and a loss of efficacy of antioxidative defenses leading to pathological states in the cells and causing tissue damage.

As it is used herein, the term "endoplasmic reticulum stress" (ERS) relates to an alteration in the function of the ER leading to the accumulation of unfolded proteins therein, inducing a state generally referred to as ER stress (ERS).

Therefore, in a first aspect, the invention relates to a method for the identification of potentially useful compounds for the treatment or the prevention of a neurodegenerative disease comprising the steps of:
a) contacting a cell with a candidate compound in any degree of purity wherein said cell expresses a DNA construct comprising:
   i) the *DDIT3* gene promoter region identified in SEQ ID NO:
      1 or a functionally equivalent variant thereof; and
   ii) a reporter gene operatively coupled to said promoter
      region
      and
b) detecting the expression of the reporter gene;
   wherein the assayed compound is identified as suitable for the treatment or prevention of said neurodegenerative disease if it causes a variation in the expression of the reporter gene in the direction opposite to the existing alteration of the levels of *DDIT3* in the neurodegenerative disease in comparison with the alteration of the expression in the absence of said compound, or if it causes an alteration in the expression of the reporter gene in the same direction as the alteration to be achieved in the levels of *DDIT3.*

Thus, the method of the invention comprises the step of detecting the expression of a gene. The person skilled in the art will note that there are conventional techniques for determining the expression levels of a certain gene in a certain cell or biological sample, such as RT-PCR, Northern blot and the like for determining the expression of the mRNA, or various types of immunoassays (Western blot, ELISA, RIA, immunoprecipitation and the like) for determining the expression of the protein.

In a preferred embodiment, the existing alteration of the levels of *DDIT3* in the neurodegenerative disease, and therefore the alteration in the expression of the reporter gene of *DDIT3,* is an increase and, accordingly, the change of expression in the reporter gene which is indicative that the candidate compound can be used for the treatment or prevention of said disease is a decrease of expression.

In a first step, the method of the invention involves contacting a cell with a candidate compound in any degree of purity. In the context of the present invention, "cell" is interpreted to mean any cell in which the *DDIT3* gene promoter identified in SEQ ID NO: 1 or a functionally equivalent variant thereof can promote the expression of a gene operatively coupled to said promoter. Generally, the expression of a gene through a promoter requires the presence of transcription factors that are able to recognize binding sequences in said promoter. Thus, the cells that can be object of the present invention include all those cells expressing the transcription factors necessary for the transcriptional activation of the promoter object of study. Although it is possible to reconstitute the expression of said reporters in cells of a very different origin, it is preferable to use cells expressing said transcription factors endogenously and constitutively. Thus, in a preferred embodiment the cells object of study include higher eukaryotic cells, preferably mammal cells. The invention contemplates the use of any type of cell in which an expression vector can be introduced. Thus, it is possible to use embryonic cells (oocytes, sperm cells, embryonic stem cells), adult cells, undifferentiated cells (fetal cells, tumor cells), differentiated cells, dividing cells, senescent cells, cells in culture and the like. In a preferred embodiment, the cell which is used in the method of the invention is a SK-N-MC cell, corresponding to a human neuroblastoma cell line.

Once the cell in which the DNA construct is going to be expressed has been selected, it is necessary to stably incorporate in said cell the DNA construct comprising the gene promoter object of study operatively coupled to a reporter gene. In the context of the present invention, "promoter" is interpreted to mean a region of DNA to which the RNA polymerase is bound and which, therefore, allows the transcription of genes which are operatively associated therewith. The present invention contemplates the use of the nucleotide sequence of the *DDIT3* gene promoter identified in SEQ ID NO: 1 as well as of a functionally equivalent variant thereof. The inventors have observed that said sequence of the *DDIT3* gene promoter identified in SEQ ID NO: 1 can be induced against endoplasmic reticulum stress. Thus, "functionally equivalent variant of SEQ ID NO: 1" is interpreted to mean all those sequences derived from the sequence of SEQ ID NO: 1 by means of modification, insertion and/or deletion of one or more nucleotides, provided that the ability to induce the expression of the gene which is operatively coupled to said sequence is substantially maintained in a situation of cell stress, particularly of the endoplasmic reticulum-type stress. "Substantially" maintaining said ability to induce the expression of said gene is interpreted to mean that said variant maintains promoter activity of at least 1.5 times the baseline expression with respect to sequence SEQ ID NO: 1. In a particular embodiment, said functionally equivalent variant is the one identified in SEQ ID NO: 2.

Polymorphism rs3847699, identified in SEQ ID NO: 2, is a single nucleotide polymorphism (SNP) in the *DDIT3* gene comprising a cytosine (C) to guanine (G) transversion in nucleotide 120 of sequence SEQ ID NO: 1. The inventors have observed that a promoter comprising the nucleotide sequence identified in SEQ ID NO: 2, can be induced both by endoplasmic reticulum stress and by oxidative stress. Thus, "functionally equivalent variant of SEQ ID NO: 2" is interpreted to mean all those sequences derived from the sequence of SEQ ID NO: 2 by means of modification, insertion and/or deletion of one or more nucleotides, provided that it maintains the previously identified polymorphism and provided that the ability to induce the expression of the gene which is operatively coupled to said sequence is substantially maintained in a situation of cell stress, particularly oxidative-type stress. In a preferred embodiment, said functionally equivalent variant is a variant responding to endoplasmic reticulum stress and oxidative stress. "Substantially" maintaining said ability to induce the expression of said gene is interpreted to mean that said variant maintains promoter activity of at least 1.5 times the baseline expression with respect to SEQ ID NO: 2.

The person skilled in the art will note that sequences functionally equivalent to the previously indicated promoter regions can be obtained provided that the essential regions within said sequences responsible for the binding of transcription factors are maintained intact. The person skilled in the art will appreciate that the binding sites in the promoter regions object of the invention include those which can be determined by means of comparison with bases from binding sites of transcription factors such as TFSEARCH (Heinemeyer, T. et al., 1998, Nucleic Acid Res., 26:364-370).

The nucleotide sequence of the *DDIT3* promoter identified in SEQ ID NO: 1 or the functionally equivalent variant thereof is operatively coupled to a reporter gene the expression of which can be easily detected. Reporter genes which can be used in the context of the present invention include luciferase, green fluorescent protein (GFP) and variants thereof emitting fluorescence at different wavelengths (for example, DS-Red or red fluorescent protein), chloramphenicol acetyltransferase, β-galactosidase, alkaline phosphatase and horseradish peroxidase. In a particular embodiment, said reporter gene is the gene encoding for luciferase.

Once the chimeric gene comprising the *DDIT3* promoter and the reporter gene is constructed, it must be introduced in a host cell. The DNA construct is introduced in the cells object of study using any of the transfection methods known for the person skilled in the art (see sections 9.1 to 9.5 in Ausubel, F.M. et al., Current Protocols in Molecular Biology, John Wiley & Sons Inc; Ringbou edition, 2003). Particularly, the cells can be transfected by means of co-precipitation of DNA with calcium phosphate, DEAE-dextran, polybrene, electroporation, microinjection, liposome-mediated fusion, lipofection, infection by retrovirus and biolistic transfection.

In a particular embodiment of the invention, said host cell temporarily expresses said DNA construct. In another particular embodiment, said cell stably expresses said DNA construct. Thus, for the purpose of obtaining stable expression of the DNA construct object of the invention, it is necessary to include in the transfection a gene encoding for resistance to a certain antibiotic, such that those cell lines which have incorporated the DNA in the genome of those cell lines in which the DNA is in an extrachromosomal position can be selected. The gene which allows selecting the cells can be provided forming part of the same vector containing the construct object of the invention or, alternatively, it can be provided separately by means of co-transfection with a second plasmid containing said resistance gene. In this latter case, the plasmid containing the DNA construct is provided to the transfection mixture in a molar excess with respect to the resistance gene such that for each resistance gene integration event there is a high probability of integration of the gene containing the promoter object of study. The plasmid containing the DNA construct is preferably provided in an excess of at least 5 times with respect to the vector containing the resistance reporter.

Suitable resistance markers for selecting cell lines which have integrated the construct in the genome include positive selection markers such as, for example, the geneticin resistance gene, the neomycin resistance gene, which confers resistance to aminoglycoside G418, the hygromycin phosphotransferase gene which confers resistance to hygromycin, the ODC gene, which confers resistance to the ornithine decarboxylase inhibitor (2-(difluoromethyl)-DL-ornithine (DFMO)), the dihydrofolate reductase gene which confers resistance to methotrexate, the puromycin-N-acetyl transferase gene, which confers resistance to puromycin, the ble gene which confers resistance to zeocin, the adenosine deaminase gene which confers resistance to 9-beta-D-xylofuranosyl adenine, the cytosine deaminase gene, which allows the cells to grow in the presence of *N*-(phosphonacetyl)-L-aspartate, thymidine kinase, which allows the cells to grow in the presence of aminopterin, the Xanthine-guanine phosphoribosyltransferase gene, which allows the cells to grow in the presence of xanthine and absence of guanine, the *E. coli* trpB gene which allows the cells to grow in the presence of indole instead of tryptophan, the *E*. *coli* hisD gene, which allows the cells to use histidinol instead of histidine. The gene of choice is incorporated in a plasmid which can additionally include a promoter suitable for the expression of said gene in eukaryotic cells (for example, the promoters CMV or SV40), an optimized translation initiation site (for example a site following the so-called Kozak rules or an IRES), a polyadenylation site such as, for example, the SV40 or phosphoglycerate kinase polyadenylation site, introns such as, for example, the beta-globulin gene intron.

The selection process of cells containing the DNA construct of interest stably integrated in the genome is carried out by means of a conventional selection process (see for example Ausubel, F.M. et al., Current Protocols in Molecular Biology (1997) 9.5.1-9.5.19). To that end, the cells are transfected with the vector or mixtures of vectors and, after a recovery period, they are allowed to grow in a selective medium (either a medium containing the antibiotic against which the reporter confers resistance or a minimum medium containing the antimetabolite against which the reporter confers resistance). The cell colonies growing in selective medium are isolated and allowed to grow again in selective medium. Once the cells which are capable of growing during repeated proliferation cycles in the presence of the selection marker have been obtained, it may be convenient to eliminate said marker from the cells, particularly if the cells are to be transfected with another selection marker. To that end, recombinases can be used, particularly the Cre/Lox system. It is alternatively possible to amplify the number of copies of the selection marker, which results in a simultaneous amplification of the number of copies of the gene of interest, with the subsequent increase in its expression. To that end, the cells are grown in the presence of progressively greater concentrations of selection agent, which results in a selection of the cells which have undergone an amplification of the genes conferring resistance to such agent and normally of the adjacent or intermediate regions. DHFR is preferably used as a selection marker and the selection of cell lines in which there is an amplification of said gene is carried out in the presence of geneticin.

A cell is normally considered to stably express a marker when the expression of said marker does not decrease with successive proliferation cycles, independently of the presence of selection agent in the culture medium.

Once a cell line which has stably integrated the DNA construct according to the invention in its genome is available, the cell is contacted with a compound or preparation the effect of which on the transcription of the reporter gene is to be studied. According to the present invention, "contacting" a cell with the candidate compound includes any possible way of taking the candidate compound inside the cell expressing the DNA construct. Thus, in the event that the candidate compound is a molecule with low molecular weight, it is enough to add said molecule to the culture medium. In the event that the candidate compound is a molecule with a high molecular weight (for example, biological polymers such as a nucleic acid or a protein), it is necessary to provide the means so that this molecule can access the cell interior. In the event that the candidate molecule is a nucleic acid, conventional transfection methods can be used, as previously described for the introduction of the DNA construct. In the event that the candidate compound is a protein, the cell can contact with the protein directly or with the nucleic acid encoding it coupled to elements allowing its transcription / translation once they are in the cell interior. To that end, any of the aforementioned methods can be used to allow its entrance into the cell interior. Alternatively, it is possible to contact the cell with a variant of the protein to be studied which has been modified with a peptide that can promote the translocation of the protein to the cell interior, such as the Tat peptide derived from the HIV-1 TAT protein, the third helix of the Antennapedia homeodomain protein from *D*. *melanogaster,* the VP22 protein of the herpes simplex virus and arginine oligomers (Lindgren, A. et al., 2000, Trends Pharmacol. Sci, 21:99-103, Schwarze, S.R. et al. , 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393).

In a particular case, the compound to be assayed is not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphorothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can access the cell interior more easily. The compounds can thus be selected based on certain parameters such as size, lipophilicity, hydrophilicity, capacity to form hydrogen bonds.

In the event that the candidate compound forms part of a more or less complex mixture, the method of the invention additionally comprises one or several steps (c) of fractionation the assayed mixture and repeating steps (a), (b) and (c) with each of the obtained fractions until the compound in the mixture which is suitable for the treatment or the prevention of the neurodegenerative disease is isolated. Methods for the fractionation of compounds present in a mixture include (thin layer, gas or gel molecular exclusion, affinity) chromatography, crystallization, distillation, filtration, precipitation, sublimation, extraction, evaporation, centrifugation, mass spectrometry, adsorption and the like. The compounds to be assayed can alternatively form part of an extract obtained from a natural source. The natural source can be an animal, plant source obtained from any environment, including, but not limited to, extracts of land, air, marine organisms and the like.

In a second step, the method of the invention includes determining the activity of the protein encoded by the reporter gene. Preferably, at the same time as the activity of the reporter gene is determined in the presence of the compounds to be assayed, it is necessary to carry out parallel determinations of the baseline transcriptional activity only in the presence of the culture medium and/or of the carrier in which the compound to be assayed is dissolved or in which the extracts to be assayed have been prepared. Generally, those compounds or extracts with transcription promoting activity will be shown because they will give values greater than 1 for the ratio between the transcriptional activity in the presence of the compound or of the candidate extract and in the presence of carrier. The compounds or extracts in which the reporter gene activity ratio is at least 1.5, 2, 3, 4, 5, 10, 20, 30, 40, 50 and up to 100 will preferably be considered positive.

The method for detecting the reporter gene expression involves contacting the cells with a compound which can generate a colored or fluorescent product in the presence of the product encoded by the reporter gene. Thus, if the enzyme is alkaline phosphatase, chromogenic substrates of the type such as p-nitrophenyl phosphate (p-NPP), 5-bromo-4-chloro 3-indolyl phosphate/nitroblue tetrazolium (BCIP/NBT), Fast-Red/naphthol-AS-TS phosphate or fluorogenic substrates of the type such as 4-methylumbelliferyl phosphate (4-MUP), 2-(5'-chloro-2'-phosphoryloxyphenyl)-6-chloro-4-(3H)-quinazolinone (CPPCQ), 3,6-fluorescein diphosphate (3,6-FDP), Fast Blue BB, Fast Red TR or diazonium salts of Fast Red Violet LB, can be used.

If the reporter gene encodes a peroxidase, chromogenic substrates of the type such as 2,2-azinobis (3-ethylbenzothiazoline-6-sulfonic acid) (ABTS), o-phenylenediamine (OPD), 3,3',5,5'-tetramethylbenzidine (TMB), o-dianisidine, 5-amino salicylic acid, 3-dimethylamino benzoic acid (DMAB) and 3-methyl-2-benzothiazoline hydrazone (MBTH), 3-amino-9-ethylcarbazole (AEC) and 3,3'-diaminobenzidine tetrahydrochloride (DAB) or fluorogenic substrates of the type such as 4-hydroxy-3-methoxyphenylacetic acid, reduced phenoxazines and reduced benzothiazines, including the reagents Amplex^{®} Red and Amplex UltraRed and reduced dihydroxanthenes, can be used.

If the reporter gene encodes a glucosidase, chromogenic substrates of the type such as o-nitrophenyl-β-D-galactoside (or-NPG), p-nitrophenyl-β-D-galactoside and 4-methylumbelliferyl-β-D-galactoside (MUG) for β-D-galactosidase and fluorogenic substrates of the type such as resorufin beta-D-galactopyranoside, fluorescein digalactoside (FDG), fluorescein diglucuronide, 4-methylumbelliferyl beta-D-galactopyranoside, carboxyumbelliferyl beta-D-galactopyranoside and coumarin beta-D-galactopyranoside, can be used. In a preferred embodiment, the reporter gene encodes for luciferase and the detection is carried out measuring the luminescence emitted by said enzyme in the presence of ATP. The luminescence is determined using commercial kits, such as for example, the enhanced luciferase assay kit (Analytical Luminescence Laboratory, MI).

In a preferred embodiment, the method of the invention is used to identify suitable compounds for the treatment or the prevention of a neurodegenerative disease wherein said disease is characterized by an increase in the expression of the *DDIT3* gene and wherein the change of expression in the reporter gene is a decrease of the expression of said *DDIT3* gene.

As has been previously mentioned, the inventors have surprisingly observed that a different capacity of response to stimuli occurs depending on the allelic variant (-1507C or - 1507G) of the *DDIT3* gene promoter analyzed. Thus, in the functional analysis of the polymorphism of the *DDIT3* promoter region, the inventors have observed a response to OS in the case of the variant -1507G (SEQ ID NO: 2) which is not observed for the variant -1507C (SEQ ID NO: 1), and a greater response to ERS in the case of the variant -1507C.

Therefore, in another aspect, the present invention relates to a method for the identification of compounds inducing endoplasmic reticulum stress in a cell comprising the steps of:
a) contacting a cell with a candidate compound in any degree of purity wherein said cell stably expresses a DNA construct comprising:
   iii) the *DDIT3* gene promoter region identified in SEQ ID NO: 1 or a functionally equivalent variant thereof; and
   iv) a reporter gene operatively coupled to said promoter
      region;
      and
b) detecting the expression of the reporter gene;
   wherein the assayed compound is identified as an inducer of said stress if it causes a variation in the expression of the reporter gene where the change is an increase of expression in comparison with the alteration of the expression in the absence of said compound.

The inventors have observed that although the *DDIT3* gene promoter region identified in SEQ ID NO: 1 responds to ERS more than the *DDIT3* gene promoter region identified in SEQ ID NO: 2, both respond to this type of cell stress. Therefore, in a particular embodiment of the invention, said functionally equivalent variant is the one identified in SEQ ID NO: 2.

In another aspect, the present invention relates to a method for the identification of compounds inducing oxidative stress in a cell comprising the steps of:
a) contacting a cell with a candidate compound in any degree of purity wherein said cell stably expresses a DNA construct comprising:
   i) the *DDIT3* gene promoter region identified in SEQ ID NO: 2 or a functionally equivalent variant thereof; and
   ii) a reporter gene operatively coupled to said promoter region
      and
b) detecting the expression of the reporter gene;
   wherein the assayed compound is identified as an inducer of said stress if it causes a variation in the expression of the reporter gene where the change is an increase of expression in comparison with the alteration of the expression in the absence of said compound.

The term "functionally equivalent variant" as it is used herein relates to any variant of the *DDIT3* gene promoter region identified in SEQ ID NO: 2 maintaining the capacity of said promoter to be induced by oxidative stress.

Likewise, in another aspect, the present invention relates to a method for the identification of compounds inhibiting endoplasmic reticulum stress in a cell comprising the steps of:
a) putting a cell in conditions of endoplasmic reticulum stress, wherein said cell stably expresses a DNA construct comprising:
   i) the *DDIT3* gene promoter region identified in SEQ ID NO:
      1 or a functionally equivalent variant thereof; and
   ii) a reporter gene operatively coupled to said promoter region;
b) contacting said cell with a candidate compound in any degree of purity; and
c) detecting the expression of the reporter gene;
   wherein the assayed compound is identified as an inhibitor of said stress if it causes a variation in the expression of the reporter gene where the change is a decrease of expression in comparison with the alteration of the expression n in the absence of said compound.

As has been previously mentioned, although the *DDIT3* gene promoter region identified in SEQ ID NO: 1 responds to ERS more than the *DDIT3* gene promoter region identified in SEQ ID NO: 2, both respond to this type of cell stress. Therefore, in a particular embodiment of the invention, said functionally equivalent variant is the one identified in SEQ ID NO: 2.

To carry out step (a) of the method, i.e., putting a cell in conditions of endoplasmic reticulum stress, the method of the invention includes any possible way of taking said cell to a condition of endoplasmic reticulum stress. As has been previously mentioned, there are various physiological or pathological situations that can affect protein folding and/or Ca²⁺ homeostasis, such as glucose deprivation, viral infections or the expression of mutant proteins unable to fold and which can be a cause of ERS. By way of a non-limiting illustration, said condition of stress is reached by means of the exposure of said cell to a protein N-glycosylation inhibitor. In a more particular embodiment, said protein N-glycosylation inhibitor is tunicamycin.

Thus, in another aspect, the present invention relates to a method for the identification of compounds inhibiting oxidative stress in a cell comprising the steps of:
a) putting a cell in conditions of oxidative stress, wherein said cell stably expresses a DNA construct comprising:
   i) the *DDIT3* gene promoter region identified in SEQ ID NO: 2 or a functionally equivalent variant thereof;
      and
   ii) a reporter gene operatively coupled to said promoter region;
b) contacting said cell with a candidate compound in any degree of purity; and
c) detecting the expression of the reporter gene;
   wherein the assayed compound is identified as an inhibitor of said stress if it causes a variation in the expression of the reporter gene where the change is a decrease of expression in comparison with the alteration of the expression in the absence of said compound.

To carry out step (a) of the method, i.e., putting a cell in conditions of oxidative stress, the method of the invention includes any possible way of taking said cell to a condition of oxidative stress. Illustrative, non-limiting examples of compounds inducing oxidative stress in a cell and which can be used in step (a) of the present method include thiol-reactive agents such as the phenylarsine oxide, sodium arsenite, mercury chloride, cadmium chloride, zinc chloride, free radical generators such as hydrogen peroxide, t-butyl hydroperoxide, cumene hydroperoxide, etc. As has been previously mentioned, reactive oxygen species (ROS) subject the organism to an oxidative stress. By way of a non-limiting illustration, said condition of stress is reached by means of the exposure of said cell to an inducer of the release of superoxide anions. In a more particular embodiment, said inducer is the xanthine/xanthine oxidase system.

The term "functionally equivalent variant" as it is used herein relates to any variant of the *DDIT3* gene promoter region identified in SEQ ID NO: 2 maintaining the capacity of said promoter to be induced by oxidative stress.

In another aspect, the present invention relates to a method for the diagnosis of a neurodegenerative disease in a subject, or for determining the predisposition of a subject for suffering a neurodegenerative disease, or for determining the stage or severity of the disease in a subject, or for monitoring the effect of the therapy administered to a subject with said disease, comprising determining the presence or absence of the polymorphism identified in SEQ ID NO: 2 of the *DDIT3* gene in a biological sample from said subject, wherein the presence of said polymorphism is indicative of said disease, or of a greater predisposition of said subject for suffering said disease, or of greater severity of the disease, or of the non-response to therapy in relation to a subject who does not present said polymorphism.

As it is used herein, the term "polymorphism" relates to a variation in the nucleotide sequence of a certain place of the DNA between the individuals of one and the same population. The polymorphism identified in SEQ ID NO: 2 is a single nucleotide polymorphism (SNP) in the *DDIT3* gene comprising a Cytosine (C) to Guanine (G) transversion. In a more particular embodiment, said polymorphism is identified with the code dbSNP rs3847699.

The detection of the polymorphism in the *DDIT3* gene in the method of the invention can be performed by means of multiple processes known by the person skilled in the art, which involve isolating the nucleic acid from a biological sample from the subject to be evaluated.

Isolating the nucleic acid of the biological sample can be performed by processes known by the person skilled in the art. Said processes can be found, for example, in Sambrook et al., 2001. "Molecular cloning: a Laboratory Manual", 3rd ed., Cold Spring Harbor Laboratory Press, N.Y., Vol. 1-3.

In addition, the present invention is not limited by the nucleic acid source, but rather the biological sample can comprise blood, saliva, amniotic fluid, tissue, etc. Thus, in a particular embodiment of the invention, the biological sample from the subject is a tissue sample comprising a nucleic acid, preferably, DNA or RNA.

After isolating the nucleic acid, the polymorphism is detected. Systems and methods for the detection of polymorphisms associated to genes include, but are not limited to, hybridization methods and array technology (e.g. technology available from Aclara BioSciences, Affymetrix, Agilent Technologies, Inc., etc), methods based on the polymerase chain reaction (e.g. TAQMAN, Applera Corp., GENECODE system, Erigen, etc.), rolling-circle amplification assays, invasive excision assays, use of mass spectroscopy, hybridization assays using a probe complementary to the polymorphism, primer extension assay, enzyme cleavage methods, NASBA, sandwich hybridization methods, methods using molecular markers, ligase chain reactions and the like. Methods for carrying out the detection of polymorphisms are described in Sambrook et *al.,* 2001 (cited *at supra*) and in patent application US2007/0105128.

In another aspect, the invention relates to a gene construct comprising a DNA sequence comprising the nucleotide sequence identified in SEQ ID NO: 1 or a functionally equivalent variant thereof. Said gene construct of the invention additionally comprises elements regulating the expression of said DNA sequence. Said regulating elements include promoters and enhancers. The promoters are typically positioned in position 5' with respect to the transcription or translation initiation site. The enhancers are capable of influencing the expression of genes when they are in position 5' or 3' with respect to the cDNA or when they form part of an intron. Regulating sequences include, in addition to promoters, sequences which facilitate the translation, processing signals for the introns, termination codons, signal sequences, internal ribosome entry sites (IRES) and polyadenylation signals.

In a particular embodiment of the invention, said functionally equivalent variant is the one identified in SEQ ID NO: 2.

Advantageously, said gene construct additionally comprises the nucleotide sequence of a gene of interest operatively linked. Said gene can be, for example, a marker or gene encoding for a motif or for a phenotype which subsequently allows the selection of a host cell transformed, transfected or infected with said construct or with an expression vector comprising said gene construct. Illustrative, non-limiting examples of said markers include antibiotic resistance genes, toxic compound resistance genes, and, generally, all those genes which allow selecting the genetically transformed cells.

Thus, in another aspect, the invention relates to an expression vector comprising a gene construct of the invention which is operatively coupled with a sequence regulating the expression of said gene construct. The person skilled in the art will note that the type of vector suitable for the expression of the nucleic acids and gene constructs of the invention will depend on the cell in which said gene construct is to be expressed. The vector of the invention can be used to transform, transfect or infect cells susceptible of being transformed, transfected or infected by said vector. Said cells can be prokaryotic or eukaryotic cells.

Therefore, in another aspect, the invention relates to a cell comprising a construct of the invention or a vector of the invention, for which said cell has been able to be transformed, transfected or infected with a construct or vector provided by this invention. Transformed, transfected or infected cells can be obtained by conventional methods known by the persons skilled in the art [Sambrok et *al.,* 1989, cited *supra*]. In a particular embodiment, said host cell is an animal cell transfected or infected with a suitable vector.

Suitable host cells include prokaryotic cells, yeasts and eukaryotic cells. In a particular embodiment, said cell is selected from a mammal cell, a plant cell, a yeast and a bacterium. The preferred host cells are eukaryotic cells. In a preferred embodiment of the invention, said cell is a cell from a SK-N-MC human neuroblastoma cell line.

In another aspect, the invention relates to a cell expressing a gene construct comprising
(i) the *DDIT3* gene promoter region comprising the sequence of SEQ ID NO: 1 or a functionally equivalent variant thereof; and
(ii) a gene of interest operatively coupled to said promoter region.

In a particular embodiment of the invention, said functionally equivalent variant is the one identified in SEQ ID NO: 2.

In another particular embodiment, said cell is a cell from a SK-N-MC human neuroblastoma cell line.

In another aspect, the invention relates to the use of a cell of the invention for the identification of suitable compounds for the treatment or the prevention of a neurodegenerative disease presenting altered levels of *DDIT3* or in which it is necessary to alter the levels of *DDIT3.* As has been previously mentioned, a cell comprising a construct or a vector of the invention can be contacted with a candidate compound in any degree of purity and, therefore, it can be used for the identification of compounds for the treatment or prevention of a neurodegenerative disease.

In a particular embodiment, said neurodegenerative disease is selected from the group consisting of Alexander disease, Alpers' disease, amyotrophic lateral sclerosis, ataxia-telangiectasia, Batten disease, spongiform encephalopathy (BSE), cocaine syndrome, corticobasal degeneration, Creutzfeldt-Jakob disease, Huntington's disease, HIV-associated dementia, Kennedy's disease, Krabbe disease, Lewy body dementia, Machado-Joseph disease, multiple sclerosis, multiple system atrophy, Neuroborreliosis, Parkinson's disease, Pelizaeus-Merzbacher disease, Pick's disease, primary lateral sclerosis, Prion diseases, Refsum's disease, Sandhoff disease, Schilder's disease, schizophrenia, Spielmeyer-Vogt-Sjogren-Batten disease, spinocerebellar ataxia, spinal muscular atrophy, Steele-Richardson-Olszewski disease, Tabes dorsalis and inflammatory cerebral disease.

The described constructs, vectors or cells of the invention can be used to obtain a transgenic non-human animal having the nucleotide sequence of the construct of the invention inserted in its genome. Thus, in another aspect, the invention relates to a transgenic animal comprising the construct of the invention.

Therefore, in another aspect, the invention relates to a transgenic non-human animal, hereinafter transgenic non-human animal of the invention, containing a construct of the invention inserted in its genome. In a particular embodiment of the invention, the transgenic non-human animal of the invention is a vertebrate, mammal, preferably a rodent, more preferably a mouse or a rat. In another particular embodiment of the invention, said non-human animal is a fish.

The transgenic non-human animal of the invention can have any genetic background of those known in the state of the art by the person skilled in the art, i.e., said transgenic non-human animal can come from a wild type (wt) animal or from a non-human animal with a genetic background incorporating any molecular marker related, directly or indirectly, with a neurodegenerative disease.

*DDIT3* is a transcription factor which is activated in response to agents inducing cell stress, particularly stresses altering the folding machinery of the ER and factors which cause oxidative stress. Therefore, said promoter can be used in the expression of a gene of interest in a regulated manner, such as an inducible protein expression system.

Thus, in another aspect, the present invention relates to a method for the regulated expression of a gene of interest comprising:
a) introducing in a cell a construct comprising the nucleotide sequence identified in SEQ ID NO: 1 or a functionally equivalent variant thereof in which said sequence is operatively coupled to said gene of interest;
b) subjecting the cell to conditions generating endoplasmic reticulum stress and, optionally,
c) recovering from the cell the product resulting from the expression of the gene of interest.

As used herein, the term "functionally equivalent variant" relates to a variant which responds or is induced under conditions of endoplasmic reticulum stress. Thus, in a particular embodiment of the invention, said functionally equivalent variant is the one identified in SEQ ID NO: 2.

In a particular embodiment, said conditions of stress are reached by means of the exposure of said cell to a protein N-glycosylation inhibitor. More particularly, said protein N-glycosylation inhibitor is tunicamycin.

In another aspect the invention relates to a method for the regulated expression of a gene of interest comprising:
a) introducing in a cell a construct comprising the nucleotide sequence identified in SEQ ID NO: 2 or a functionally equivalent variant thereof in which said sequence is operatively coupled to said gene of interest,
b) subjecting the cell to conditions generating oxidative stress and, optionally,
c) recovering from said cell the product resulting from the expression of the gene of interest.

In a particular embodiment of the invention, said conditions of stress are reached by means of the exposure of said cell to an inducer of the release of superoxide anions in the cell. In a preferred embodiment, said inducer is the xanthine/xanthine-oxidase system.

The present invention is additionally explained below by means of examples. In no case must it be interpreted as a limitation of the scope of the invention as it is defined in the claims.

### EXAMPLE 1

### I. MATERIALS AND METHODS

### 1. Plasmids and cloning

The *DDIT3* promoter amplified by PCR is subcloned into pcDNA3, from Invitrogen and is subsequently cloned into the plasmid pXP2 which has no promoter activity per se (Nordeen, S.K. (1988) "Luciferase reporter gene vectors for analysis of promoters and enhancers." Biotechniques 6(5): 454-8). The plasmid which was used as control in the transient transfection experiments was the construct pSV-β-galactosidase, from Promega.

### 1.1. Cloning the DDIT3 gene promoter region (variant -1507 C) in a plasmid containing a reporter gene

For the *DDIT3* gene, the region corresponding to nucleotides -1626 to +60 , taking the start of the transcription (nucleotides 20059232 to 20027547 of the reference sequence NT_029419 from the NCBI database) as +1, was amplified by PCR from genomic DNA of an individual who had the allelic form - 1507C. Said amplified *DDIT3* gene promoter region corresponds to the nucleotide sequence identified in SEQ ID NO: 1.

The oligonucleotides for the PCR (Invitrogen) include respective restriction targets in order to facilitate the cloning by creating after the restriction respective sticky ends (indicated as underlined text) and tails to facilitate the cleavage by the restriction enzyme of the target (in lower case letters):
Chop-PromU: 5' cta gta gct tgg atC CGG CCT TGT GAC AGT TTC T 3' (SEQ ID NO: 3)
Chop-PromL: 5' tat aca cta caa gct TGC CAC CCG CTC ATC TTT 3' (SEQ ID NO: 4)

The following PCR conditions (reagents from Biotools, except the nucleotides, from Applied Biosystems) were used: 50 ng of genomic DNA were incubated with 75 mM Tris HCl, pH 9.0, 1 mM Cl₂Mg, 1 unit of Taq polymerase, 200 µM nucleotides and 0.8 µM oligonucleotides, in a PCR program which included an initial denaturation at 94°C 5 minutes, 40 cycles of 94°C 30 seconds, 55°C 30 seconds and 72°C 1 minute and a final elongation of 72°C 10 minutes, in the Perkin Elmer *GeneAmp PCR System 9600* thermal cycler.

The amplified products were purified with the Qiagen QIAquick^{®} PCR Purification kit, they were subjected to enzyme digestion for 16 hours at 37°C in buffer 2 (New England Biolabs) with 10 units of *BamH*I (New England Biolabs) and of *Hind*III *(DDIT3* promoter) and these digested amplified products were band purified with the Qiagen QIAquick^{®} Gel Extraction kit.

The digested and purified promoter was subsequently inserted in the plasmid pcDNA3, which had been previously digested and purified in the same manner as the corresponding promoter, using the Quick Ligation Kit, from New England Biolabs, according to the instructions recommended by the manufacturer, 10 minutes at room temperature.

XL1-Blue competent bacteria were transformed by means of heat shock (42°C 30 seconds), and they were grown in selective medium (LB: 5 grams of yeast extract, 10 grams of Tryptone-Peptone -both from Difco- and 10 grams of NaCl -Merck- per liter, with 100 µg/ml ampicillin -Roche-). The plasmid DNA was extracted from the bacteria transformed using the Promega Wizard^{®} Plus SV Minipreps kit.

To subclone the promoter region into pXP2, it was extracted by means of restriction from pcDNA3 with the same conditions and restriction enzymes used in the previous steps, it was band purified and ligated in pXP2, also digested and purified in the already described manner. Competent bacteria were transformed and the plasmid DNA was extracted therefrom by the already described method.

Once the sequence was verified, MaxiPreps were performed with the QIAGEN® Plasmid Purification kit, from Qiagen, according to the manufacturer's indications, to obtain an amount of plasmid enough for the subsequent cell transfections.

The plasmid pXP2 has the cloning site in 5' at the start of the luciferase gene encoding sequence. This enzyme catalyzes a reaction which, in the presence of luciferin, generates photon emission. The luciferase activity can thus be measured in an illuminometer quantifying the emitted flash, and thus, the measurement of the luciferase activity is a quantitative indicator of the transcriptional activity of the promoter region cloned into the plasmid pXP2, directing its expression.

### 1.2. Generation of the variant -1507G by means of directed mutagenesis

The other allelic variant, hereinafter variant -1507G, was obtained by directed mutagenesis with the Stratagene QuickChange® Site-directed Mutagenesis kit, according to the manufacturer's indications. The sequence of the oligonucleotides used is indicated below, indicating the base to be mutated with underlining (manufactured by Invitrogen):
Ddit3-MutU: 5'ACT TTG CAT TCT GGG TGG TGC GTT TT 3'(SEQ ID NO: 5)
Ddit3-MutL: 5'AAA ACG CAC CAC CCA GAA TGC AAA GT 3' (SEQ ID NO: 6)

The PCR program consisted of an initial denaturation at 95°C for 30 seconds and 16 cycles of 95°C 30 seconds and 68°C 10 minutes in the same thermal cycler where the PCR was performed. The PCR product was subsequently incubated at 37°C for 1 hour with *DpnI* to eliminate the original plasmid.

Competent bacteria were transformed and the plasmid DNA was purified in the same manner already described for the first allelic variant.

The sequence of all the constructs generated was verified in the sequencing service of the Parque Cientifico de Madrid (PCM).

### 2. Cell lines

The SK-N-MC human neuroblastoma cell line (American Type Culture Collection, ATCC, ref. HTB-10) was used. It was grown in monolayer on culture plates with a diameter of 10 cm (P100, from Falcon) and maintained in minimal essential medium (MEM -Gibco), supplemented with 10% fetal bovine serum (Sigma) decomplementing for 30 minutes at 56°C, non-essential amino acids (39.2 mg/l L-alanine, 60 mg/l L-asparagine·H₂O, 53.2 mg/l L-aspartic acid, 58.8 mg/l L-glutamic acid and 46 mg/l L-proline, all from Merck), 4 mM L-glutamine -Merck-,1 mM sodium pyruvate -Merck-, and 50 µg/ml gentamicin -Sigma-.

The cells were cultured at 37°C in a humidified atmosphere with 7% CO₂.

### 3. Transfection of cells and measurement of the transcriptional activity

The collection of generated plasmids was used for the transient and stable transfection of SK-N-MC human neuroblastoma cells.

### 3.1. Transient transfection with the constructs pXP2-DDIT3

On the day before the transfection 2 x 10⁵ cells per well are seeded in a 24-well plate (M24, from Falcon). 0.7 µg of DNA per well are transfected mixing the plasmids pXP2-DDIT3 and pSV-β-galactosidase in a 1:1 ratio (0.35 µg of pXP2 and 0.35 µg of β-gal) with Lipofectamine Plus (from Life Technologies), following the manufacturer's indications, in a 1:3 DNA:Lipofectamine ratio and allowing the cells to incubate with the plasmid for 5 hours. Twenty-four hours after the transfection the cells are treated to see the response to the stimulus.

To analyze the cells transfected with constructs expressing luciferase, after washing with Phosphate buffered saline (PBS) (8 g/l NaCl, 0.2 g/l KCl, 2.89 g/l HNa₂(PO₄)₂·12 H₂O and 0.2 g/l HK(PO₄), all from Merck), the cells are lysed with 200 µl of the Promega Cell Culture Lysis Reagent kit, (25 mM Tris pH 7.8 with H₃PO₄, 2 mM CDTA, 2 mM DTT, 10% glycerol and 1% Triton X-100). To facilitate the lysis, they are left for 10 minutes at room temperature and then the cells were subjected to a cycle of freezing (at least 2 hours at -70°C) and thawing at room temperature; the samples were centrifuged at 13,000 rpm for 10 minutes to discard the large cell remains.

The cell lysate was assayed for the β-galactosidase activity, as an indicator of the transfection efficiency, and luciferase activity, as an indicator of the transcriptional activity of the promoters:
- Assay of β-galactosidase activity. 50 µl of cell lysate are incubated in an E.L.I.S.A. plate with 50 µl of enzyme substrate (2 mM MgCl₂ -Merck-, 100 mM β-mercaptoethanol -Sigma-, 120 mM Na₂HPO₄, 80 mM NaH₂PO₄ - both from Carlo Erba- and 1.33 mg/ml of ONPG (2-Nitrophenyl-β-D-galactopyranoside) -Sigma-). This reaction generates a colored product the concentration of which is measured as absorbance at 415 nm in an E.L.I.S.A. plate reader (Model 680, from BioRad).
- Assay of luciferase activity. 40 µl of cell lysate are measured adding 100 µl of reagent of the enzyme (20 mM Tricin, 1.07 mM MgCO₄, 33.3 mM DTT, 270 µM Coenzyme A -all from Sigma-, 2.67 mM MgSO₄, 0.1 mM EDTA
   -both from Merck-, 470 µM Luciferin potassium salt - Promega- and 530 µM ATP -Boehringer Mannheim-);
   alternatively, the one of the Luciferase Assay System kit -Promega- was used. The luminescence emission is recorded in a Monolight 2010 illuminometer (Analytical Luminescence Laboratory).

The transcriptional activity of the different constructs is expressed as the relative luciferase/β-galactosidase activity.

### 3.2. Stable transfection with the construct of pXP2-DDIT3

Two days before the transfection, 3 x 10⁶ cells are seeded in a P100 culture plate. The plasmids pXP2-DDIT3 (constructs - 1507C or -1507G) and pcDNA3 are mixed in a 90:10 ratio to transfect 16 µg of DNA (14.4 µg of pXP2 and 1.6 µg of pcDNA3) per plate with Lipofectamine Plus, according to the manufacturer's indications, in a 1:4 DNA:Lipofectamine ratio for 5 hours.

The cells are collected from the P100 plate by means of trypsinization with a solution of 500 mg/l trypsin (Disco) and 160 mg/l EDTA (Merck) and 1/2, 1/10, 1/100 and 1/1000 transfection dilutions are seeded. On the following day, and after that every 3 or 4 days (twice a week), the medium is substituted with fresh medium with 400 µg/ml Geneticin -Gibco- to obtain isolated clones.

The clones resistant to the antibiotic are isolated by aspiration with an automatic pipette and seeded in an M24 for the expansion thereof. The expression of luciferase is analyzed and the lines having greater luciferase activity are maintained. Furthermore, a standardization of the amount of construct pXP2 is performed according to the number of cells. To that end, the cell DNA of half the volume resulting from the lysis is purified with the High Pure PCR Template Preparation kit and real time PCRs are performed in the AbiPrisrism^{®} 7900HT Sequence Detection System apparatus, from Applied Biosystems in the universal PCR conditions indicated by the manufacturer: the amount of plasmid in the extract is determined by amplifying a fragment of the luciferase gene with the oligonucleotides:
5'CCT TGA TTG ACA AGG ATG GAT GGC 3' (SEQ ID NO: 7) and
5'CAT CGT CGG GAA GAC CTG CCA CGC CC 3' (SEQ ID NO: 8)
   and its relative amount is calculated according to the number of cells by means of the Applied Biosystems Hs99999901_s1 assay, to quantify the amount of 18S, constant in the cell.

### 4. Cell treatments

To induce oxidative stress (OS), the Xanthine/Xanthine-Oxidase (XXO) system (10 µM Xanthine -Sigma- in 0.1 mM NaOH - Merck- and 50 mU/ml Xanthine-Oxidase -Roche-), which causes release of superoxide anions (O²⁻) is used.

To induce endoplasmic reticulum stress (ERS), Tunicamycin (Tn) (2.5 µg/ml in DMSO, all from Sigma), which is an antibiotic produced by *Streptomyces lysosuperficus* which inhibits protein N-glycosylation is used.

For the convergence of both stresses (ISR), XXO and Tn are added simultaneously in the amounts already indicated.

The treatments were punctually performed, collecting at a certain point (normally, 18 hours), or in kinetics, collecting points for 24 hours at 3-hour intervals.

### II. RESULTS

### 1. Obtaining the plasmid constructs used

An amplified product encompassing the region between positions -1626 to +60 *(DDIT3* promoter, allelic form -1507C), taking position +1 in the start of the transcription, was cloned into the expression vector pcDNA3. The promoter region was subsequently extracted by restriction from pcDNA3 to subclone them into the vector pXP2.

A plasmid containing the *DDIT3* promoter bound in 5' to the luciferase encoding sequence was thus obtained. This construct was used to perform thereon the directed mutagenesis and thus obtain the other allelic variant: -1507G *(DDIT3* promoter).

The plasmids were sequenced in the PCM, determining that the promoter sequence coincides with the one published with accession number NT_029419 from the NCBI database, nucleotides from 20059232 to 20057547.

### 2. Obtaining two cell lines stably expressing the luciferase under the control of the DDIT3 promoter

The SK-N-MC human neuroblastoma cell line was stably cotransfected with the pXP2 plasmid construct with the already described *DDIT3* promoter (a construct for each allele from - 1507, C and G), together with pcDNA3, which confers resistance to the antibiotic Geneticin, in order to select the cell clones which have acquired the plasmids.

Sixteen colonies resistant to Geneticin were isolated and amplified, and their baseline expression of luciferase was analyzed. In the clones for which luciferase counts of at least order 10⁴ per assay volume were obtained, their load of the construct of plasmid pXP2 standardized according to the number of cells and also the expression of luciferase in baseline conditions and in response to stimuli were analyzed. The results obtained for each cell clone are detailed in Table 1.

**Table 1. Analysis of the clones stably transfected with the constructs in pXP2 DDIT3(-1507C) - Luciferase and DDIT3(-1507G) - Luciferase. For each allelic variant of the position -1507 of the DDIT3 promoter, the luciferase counts obtained in 10% of cell extract from an M24 well at 80% confluence and, in the event of being determined, the amount of copies relative to the amount of 18S of the extract are indicated in each analyzed clone (nd, not determined).**

| CLONE | -1507C Luciferase (counts) | % pXP2 / 18S copies | CLONE | -1507G Luciferase (counts) | % pXP2 / 18S copies |
|---|---|---|---|---|---|
| C1 | 69,198 | 6.05 | G1 | 1,841 | nd |
| C2 | 69,198 | 4.66 | G2 | 25,168 | 82.73 |
| C3 | 70,883 | 3.31 | G3 | 64,915 | 8.36 |
| C4 | 147,104 | 3.46 | G4 | 318,561 | 24.90 |
| C5 | 145 | nd | G5 | 112,015 | 34.74 |
| C6 | 145,213 | 3.77 | G6 | 218 | nd |
| C7 | 222,361 | 17.25 | G7 | 62,890 | 6.97 |
| C8 | 4,778 | nd | G8 | 3,240 | nd |

The characterization of the colonies C1 (allele -1507C) and G7 (allele -1507G) was performed since they had a similar cell growth and plasmid load.

### 3. Study of the transcriptional activity of the DDIT3 promoter

The plasmids with the *DDIT3* promoter region were used in transient and stable transfection experiments in SK-N-MC cells.

The cells were transfected using lipofectamine, which forms complexes with the plasmids, and which are what the cells capture. They were subsequently treated according to models fine-tuned in the laboratory to simulate situations of (oxidative and/or reticulum) stress for enough time to allow the cells to express luciferase, which is the activity which is assayed to determine the transcriptional activity of the promoters cloned in response to these stimuli.

### 3.1. In transiently transfected cells

Punctual and kinetic experiments of different culture conditions in SK-N-MC cells transiently expressing the luciferase enzyme under the regulation of the *DDIT3* promoter with the allelic variants C and G in position -1507 were performed.

The transcriptional activity of the two constructs in the different culture conditions over time is shown in Figure 1. The Luciferase Counts (x10⁻³) are represented per milliunit of β-galactosidase; points were collected for the analysis thereof every 3 hours until a final time of 24.

In response to the different culture conditions it is observed that for both allelic variants, there is a slight stimulation of the transcriptional activity in response to the culture conditions of OS and a more pronounced activation in response to the conditions of ERS and of ISR.

In view of these data, it can be stated that the cloned promoter region contains the minimum elements necessary to maintain the capacity of response of the *DDIT3* gene to stress, therefore the two plasmids generated (pXP2 *DDIT3*(-1507C) and pXP2 *DDIT3*(-1507G)) are useful for assaying the activation of the *DDIT3* gene and, indirectly, due the fact that this gene is an indicator of the induction of cell death by apoptosis, for assaying the neurotoxic or neuroprotective activity of compounds.

### 3.2. In stably transfected cells (Stable cell lines C1 (-1507 C) and G7 (-1507 G)

Punctual and kinetic experiments of different culture conditions in SK-N-MC cells stably expressing the luciferase enzyme under the regulation of the *DDIT3* promoter with the allelic variants C and G in position -1507 (lines C1 and G7, respectively) were performed.

The luciferase activity counts were standardized with respect to the plasmid load per cell and with respect to the number of cells as has been previously described. It was verified that the plasmid load was the same for both cell lines and that it was not altered by the treatments used.

The results obtained for a treatment of 24 hours collecting points every 3 hours, expressed in standardized luciferase activity, which measures the transcriptional activity of the *DDIT3* promoter for both allelic variants (-1507 C and G) during the treatment, are shown in Figure 2.

It is observed that in baseline conditions the transcriptional activity remains virtually constant throughout the entire study, without observing statistically significant differences between both variants. Broadly speaking, a more pronounced stimulation in response to the culture conditions of ERS and of ISR is observed for both cell lines.

The detailed analysis of the behavior of the lines in response to the different stimuli showed that:

### C1 cell line (DDIT3 -1507C) (Figure 2, panel A).

- In conditions of OS, the transcriptional activity of the promoter remains virtually constant over time, without observing statistically significant differences with respect to that observed in baseline conditions.
- In conditions of ERS, a statistically significant progressive increase of the transcriptional activity is started to be observed after 6 hours of treatment, until reaching maximum values after about 15-18 hours of treatment.
- In conditions of ISR, the transcriptional activity is similar to that already described in conditions of ERS, but with levels of transcriptional activity slightly lower in ISR than in ERS.

### G7 cell line (DDIT3 -1507G) (Figure 2, panel B).

- In conditions of OS, a transcriptional activity in response to the stimulus greater than the activity observed in baseline conditions can be observed after 9 hours of treatment, with a maximum activity after 15 hours.
- In conditions of ERS, a statistically significant progressive increase over time of the transcriptional activity is also started to be observed after 6 hours of treatment, until reaching a maximum after about 15 hours. In the case of this allelic variant, the maximum level of transcriptional activity does not reach the levels reached by the variant -1507C.
- In conditions of ISR, the transcriptional activity profile is similar to that already described in conditions of ERS, but observing a greater response, such that after 15 hours of treatment the difference between these two responses (ISR and ERS) is statistically significant.

In short, this study shows a difference of behavior of the alleles C and G in response to OS, as well as to the conditions of ISR with respect to those of ERS. To quantify this difference, the data corresponding to 15 hours of culture were used, at which time the transcriptional activity reached the maximum in all the cultures, although this different behavior is maintained for all the times greater than this time. The results of the comparison, expressing the transcriptional activity in times with respect to the baseline conditions of culture, are shown in Table 2.

**Table 2. Luciferase activity of the cells stably transfected with the constructs pXP2 - DDIT3 - Luciferase (-1507C or -1507G) after 15 hours of treatment, with respect to the non-treated cells, in times ± the standard error of the mean.**

| Activation of the *DDIT3* promoter by Oxidative stress (OS), Endoplasmic reticulum stress (ERS) and their combination (ISR) | | |
|---|---|---|
| OS | -1507C | -1507G |
| | 1.1 ± 0.2 | 1.8 ± 0.2 * |
| ERS | 4.6 ± 0.4 | 3.4 ± 0.3 * |
| ISR | 4.1 ± 0.4 | 4.3 ± 0.4 |

| | | |
|---|---|---|
| (*) Significant with respect to the allele C (Student's t p<0.05). | | |

### DISCUSSION

### Role of the polymorphism of the DDIT3 gene promoter on the pathogenesis of Alzheimer's disease.

In the functional analysis of the polymorphism of the *DDIT3* promoter region, the inventors have observed a different capacity of response to stimuli depending on the allelic variant analyzed. The experimental results indicate a response to OS in the case of the variant -1507G which is not observed for the variant -1507C, which leads to a greater transcriptional activity of the promoter with this polymorphic variant in response to OS, which could confer to the cells a greater predisposition to the apoptosis directed by this gene in conditions of OS.

This fact could explain the risk in elderly individuals with AD associated with the fact of being carriers of the allele G in this polymorphic site, because they are individuals in whom, due to the actual aging process, there is an intrinsic OS generating transcriptional levels greater than in the case of the protective allele, C. This could occur if there is a transcription activating factor which is activated in response to OS and which is exclusively bound to the polymorphic variant -1507G.

In relation to the difference observed in response to ERS, although both variants have capacity of transcriptional response, the latter is greater in the case of -1507C, in which maximum transcriptional values are reached. Levels of ERS high enough to lead the cells to apoptosis are present in individuals with overload of ER, as could be the case of individuals with mutations. In this case, the functional alteration caused by the mutation and/or the overload of endoplasmic reticulum would be strong enough to reach levels of Chop leading the cells to apoptosis.

This overexpression of *DDIT3* in response to ERS could explain the observed effect of interaction between the polymorphism and the age of onset of the symptoms: in the youngest mature individuals there would be an overload of ER leading to ERS, but the OS due to aging has still not occurred. Thus, the individuals homozygous for the allelic variant -1507C would have expression levels of *DDIT3* greater than those of the individuals carrying the variant -1507G, directing the cells towards apoptosis, thus bringing forward the age of onset of the symptoms of the disease.

In response to ISR the transcriptional levels are equal in both allelic variants, maximum transcriptional levels being obtained. As it refers to a convergence of oxidative and ER stresses, they are probably acting as multiple transcription factors which, in their entirety, mask the differential effect in the transcriptional activity depending on the allelic variant presented.

### Functionality of the allelic variants -1507 C and G of the DDIT3 gene promoter

For the purpose of analyzing if this polymorphism could cause differences in the expression level of the gene, transient transfection assays were performed in SK-N-MC cells and stable transfecting lines were generated, and the expression was studied in baseline culture conditions and in response to inducers of stress related to neurodegeneration and AD.

### In transient transfection assays

The obtained results indicate that all the stimuli to which the cells have been subjected allow seeing an alteration of the promoter activity after 6 hours of treatment; specifically, the transcriptional activity observed after the treatments with the Xanthine/Xanthine Oxidase system, which generates OS, with tunicamycin, to produce ERS, and with both treatments jointly, which generates ISR, increases significantly with a behavior similar to that observed for the endogenous *DDIT3* promoter.

In addition, the two allelic variants have a behavior similar in these assays.

### In the stable transfecting neuronal lines

It is observed that these lines behave in a similar manner to that described in the transient transfections but, unlike what occurred there, a differential behavior here according to the allele providing the stable cell line is observed here:
Although the activity in baseline culture conditions is not different therebetween, the response to the stimuli is different. Specifically, the variant -1507G seems to respond less to ERS and more to OS than the variant -1507C. This fact could be due to there being a binding site for transcription factors related to the response to stimuli in the position where polymorphism rs3847699 is located. In fact, with transcription sites prediction programs, it is found that the human transcription factors RREB-1 and LBP-1c/CP2/LSF (CP2) could bind to the region between the positions from -1500 to -1519 and from - 1505 to -1515, respectively, but only in the event that the allelic variant present is G. The transcription factor CP2 (*TFCP2* gene, location 12q13) has been related to AD, insofar as an association between a polymorphism (A/G) of the 3'UTR region of the gene and the disease has been described. It has been seen that the 3'UTR region binds to proteins, such that the allele A, protective against AD, has a weaker binding than the allele G. This factor regulates the expression of other genes which have been already related to the AD (GSK3, α-2macroglobulin, NFκB, interleukin 1 or TNF), it can modulate the activation of various viruses, such as HSV-1, and interacts with the protein Fe65, which regulates the internalization and the processing of APP.

It is possible that the polymorphism studied in the *DDIT3* promoter region has, as suggested by the data shown herein, a repercussion in the capacity of response of this gene to various stimuli because said polymorphism affects the binding of CP2 and other transcription factors, and that the differential response to stress-inducing stimuli is responsible for the association with the age of onset of Alzheimer's disease observed by the inventors.

The response to stimuli of the two cell lines generated, as well as their differential response to oxidative stress (OS) and to endoplasmic reticulum stress (ERS) indicates that these two lines (and the plasmids used to generate them) are very useful tools for assaying the capacity of compounds to: activate the *DDIT3* gene, induce or inhibit oxidative stress and endoplasmic reticulum stress, as well as induce or inhibit neuronal death by apoptosis (neuroprotection assays).

## Claims

1. Method for the identification of potentially useful compounds for the treatment or the prevention of a neurodegenerative disease comprising the steps of:
a) contacting a cell with a candidate compound in any degree of purity, wherein said cell stably expresses a DNA construct comprising:
i) the *DDIT3* gene promoter region identified in SEQ ID NO:
1 or a functionally equivalent variant thereof; and
ii) a reporter gene operatively coupled to said promoter region
and
b) detecting the expression of the reporter gene;
wherein the assayed compound is identified as suitable for the treatment or prevention of said neurodegenerative disease if it causes a variation in the expression of the reporter gene in the direction opposite to the existing alteration of the levels of *DDIT3* in the neurodegenerative disease in comparison with the alteration of the expression in the absence of said compound or if it causes an alteration in the expression of the reporter gene in the same direction as the alteration to be achieved in the levels of *DDIT3.*

2. A method according to claim 1, wherein said variant is the one identified in SEQ ID NO: 2.

3. A method according to claim 1 or 2, wherein said neurodegenerative disease presents an increase in the expression levels of *DDIT3,* and wherein the change of expression in the reporter gene which is detected in step (b) is a decrease of expression.

4. A method according to any of claims 1 to 3, wherein said neurodegenerative disease is selected from the group consisting of Alzheimer's disease, Alexander disease, Alpers' disease, amyotrophic lateral sclerosis, ataxia-telangiectasia, Batten disease, bovine spongiform encephalopathy (BSE), cocaine syndrome, corticobasal degeneration, Creutzfeldt-Jakob disease, Huntington's disease, HIV-associated dementia, Kennedy's disease, Krabbe disease, Lewy body dementia, Machado-Joseph disease, multiple sclerosis, multiple system atrophy, Neuroborreliosis, Parkinson's disease, Pelizaeus-Merzbacher disease, Pick's disease, primary lateral sclerosis, prion diseases, Refsum's disease, Sandhoff disease, Schilder's disease, schizophrenia, Spielmeyer-Vogt-Sjogren-Batten disease, spinocerebellar ataxia, spinal muscular atrophy, Steele-Richardson-Olszewski disease, Tabes dorsalis and inflammatory cerebral disease.

5. A method according to any of claims 1 to 4, wherein said cell is a SK-N-MC cell.

6. A method according to any of claims 1 to 5 wherein if the candidate compound forms part of a mixture, the method additionally comprises one or several steps (c) of fractionating the assayed mixture and repeating steps (a), (b) and (c) with each of the obtained fractions until the compound in the mixture which is suitable for the treatment or the prevention of the neurodegenerative disease is isolated.

7. A method for the identification of compounds inducing endoplasmic reticulum stress in a cell comprising the steps of:
a) contacting a cell with a candidate compound in any degree of purity wherein said cell stably expresses a DNA construct comprising:
i) the *DDIT3* gene promoter region identified in SEQ ID NO:
1 or a functionally equivalent variant thereof; and
ii) a reporter gene operatively coupled to said promoter region
and
b) detecting the expression of the reporter gene;
wherein the assayed compound is identified as an inducer of said stress if it causes a variation in the expression of the reporter gene where the change is an increase of expression in comparison with the alteration of the expression in the absence of said compound.

8. A method according to claim 7, wherein said variant is the one identified in SEQ ID NO: 2.

9. A method for the identification of compounds inducing oxidative stress in a cell comprising the steps of:
a) contacting a cell with a candidate compound in any degree of purity wherein said cell stably expresses a DNA construct comprising:
i) the *DDIT3* gene promoter region identified in SEQ ID NO: 2 or a functionally equivalent variant thereof; and
ii) a reporter gene operatively coupled to said promoter region
and
b) detecting the expression of the reporter gene;
wherein the assayed compound is identified as an inducer of said stress if it causes a variation in the expression of the reporter gene where the change is an increase of expression in comparison with the alteration of the expression in the absence of said compound.

10. A method for the identification of compounds inhibiting endoplasmic reticulum stress in a cell comprising the steps of:
a) putting a cell in conditions of endoplasmic reticulum stress, wherein said cell stably expresses a DNA construct comprising:
i) the *DDIT3* gene promoter region identified in SEQ ID NO:
1 or a functionally equivalent variant thereof; and
ii) a reporter gene operatively coupled to said promoter region;
b) contacting said cell with a candidate compound in any degree of purity; and
c) detecting the expression of the reporter gene;
wherein the assayed compound is identified as an inhibitor of said stress if it causes a variation in the expression of the reporter gene where the change is a decrease of expression in comparison with the alteration of the expression in the absence of said compound.

11. A method according to claim 10, wherein said variant is the one identified in SEQ ID NO: 2.

12. Method according to any of claims 10 to 11, wherein said conditions of stress are reached by means of the exposure of said cell to a protein N-glycosylation inhibitor, to thapsigargin or to a temperature drop.

13. Method according to claim 12, wherein said protein N-glycosylation inhibitor is tunicamycin.

14. A method for the identification of compounds inhibiting oxidative stress in a cell comprising the steps of:
a) putting a cell in conditions of oxidative stress, wherein said cell stably expresses a DNA construct comprising:
i) the *DDIT3* gene promoter region identified in SEQ ID NO: 2 or a functionally equivalent variant thereof; and
ii) a reporter gene operatively coupled to said promoter region;
b) contacting said cell with a candidate compound in any degree of purity; and
c) detecting the expression of the reporter gene;
wherein the assayed compound is identified as an inhibitor of said stress if it causes a variation in the expression of the reporter gene where the change is a decrease of expression in comparison with the alteration of the expression in the absence of said compound.

15. Method according to claim 14, wherein said conditions of stress are reached by means of the exposure of said cell to an inducer of the release of superoxide anions or of free radicals in the cell.

16. The method according to claim 15, wherein said inducer is the xanthine/xanthine-oxidase system or hydrogen peroxide.

17. A method for the diagnosis of a neurodegenerative disease in a subject, or for determining the predisposition of a subject for suffering a neurodegenerative disease, or for determining the stage or severity of the disease in a subject, or for monitoring the effect of the therapy administered to a subject with said disease, comprising determining the presence or absence of the polymorphism identified in SEQ ID NO: 2 of the *DDIT3* gene in a biological sample from said subject, wherein the presence of said polymorphism is indicative of said disease, or of a greater predisposition of said subject for suffering said disease, or of greater severity of the disease, or of the non-response to therapy in relation to a subject who does not present said polymorphism.

18. The method according to claim 17, wherein said neurodegenerative disease is selected from the group consisting of Alzheimer's disease, Alexander disease, Alpers' disease, amyotrophic lateral sclerosis, ataxia-telangiectasia, Batten disease, bovine spongiform encephalopathy (BSE), cocaine syndrome, corticobasal degeneration, Creutzfeldt-Jakob disease, Huntington's disease, HIV-associated dementia, Kennedy's disease, Krabbe disease, Lewy body dementia, Machado-Joseph disease, multiple sclerosis, multiple system atrophy, Neuroborreliosis, Parkinson's disease, Pelizaeus-Merzbacher disease, Pick's disease, primary lateral sclerosis, prion diseases, Refsum's disease, Sandhoff disease, Schilder's disease, schizophrenia, Spielmeyer-Vogt-Sjogren-Batten disease, spinocerebellar ataxia, spinal muscular atrophy, Steele-Richardson-Olszewski disease, Tabes dorsalis and inflammatory cerebral disease.

19. A gene construct comprising a DNA sequence comprising the nucleotide sequence identified in SEQ ID NO: 1 or a functionally equivalent variant thereof.

20. Gene construct according to claim 19, wherein said variant presents the polymorphism identified in SEQ ID NO: 2.

21. A gene construct according to claim 19 or 20, additionally comprising the operatively linked nucleotide sequence of a gene of interest.

22. An expression vector comprising a gene construct according to any one of claims 19 to 21.

23. A cell comprising a gene construct according to any one of claims 19 to 21, or an expression vector according to claim 22.

24. A cell expressing a gene construct comprising
a) the *DDIT3* gene promoter region comprising the sequence of SEQ ID NO: 1 or a functionally equivalent variant thereof; and
b) a gene of interest operatively coupled to said promoter region.

25. The cell according to claim 24, wherein said variant is the one identified in SEQ ID NO: 2.

26. The cell according to claims 23 to 25 wherein said cell is a SK-N-MC cell.

27. Use of a cell according to any one of claims 23 to 26 for the identification of suitable compounds for the treatment or the prevention of a neurodegenerative disease presenting altered levels of *DDIT3,* or in which it is necessary to alter the levels of *DDIT3.*

28. Use according to claim 27, wherein said neurodegenerative disease is selected from the group consisting of Alzheimer's disease, Alexander disease, Alpers' disease, amyotrophic lateral sclerosis, ataxia-telangiectasia, Batten disease, bovine spongiform encephalopathy (BSE), cocaine syndrome, corticobasal degeneration, Creutzfeldt-Jakob disease, Huntington's disease, HIV-associated dementia, Kennedy's disease, Krabbe disease, Lewy body dementia, Machado-Joseph disease, multiple sclerosis, multiple system atrophy, Neuroborreliosis, Parkinson's disease, Pelizaeus-Merzbacher disease, Pick's disease, primary lateral sclerosis, prion diseases, Refsum's disease, Sandhoff disease, Schilder's disease, schizophrenia, Spielmeyer-Vogt-Sjogren-Batten disease, spinocerebellar ataxia, spinal muscular atrophy, Steele-Richardson-Olszewski disease, Tabes dorsalis and inflammatory cerebral disease.

29. Method for the regulated expression of a gene of interest comprising
a) introducing in a cell a construct comprising the nucleotide sequence identified in SEQ ID NO: 1 or a functionally equivalent variant thereof in which said sequence is operatively coupled to said gene of interest;
b) subjecting the cell to conditions generating endoplasmic reticulum stress and, optionally,
c) recovering from the cell the product resulting from the expression of the gene of interest.

30. Method according to claim 29, wherein said variant is the one identified in SEQ ID NO: 2.

31. Method according to any of claims 29 to 30, wherein said conditions of stress are reached by means of the exposure of said cell to a protein N-glycosylation inhibitor.

32. Method according to claim 31, wherein said protein N-glycosylation inhibitor is tunicamycin.

33. Method for the regulated expression of a gene of interest comprising
a) introducing in a cell a construct comprising the nucleotide sequence identified in SEQ ID NO: 2 or a functionally equivalent variant thereof in which said sequence is operatively coupled to said gene of interest,
b) subjecting the cell to conditions generating oxidative stress and, optionally,
c) recovering from said cell the product resulting from the expression of the gene of interest.

34. Method according to claim 33, wherein said conditions of stress are reached by means of the exposure of said cell to an inducer of the release of superoxide anions in the cell.

35. Method according to claim 34, wherein said inducer is the xanthine/xanthine-oxidase system.

36. Method according to any of claims 33 to 35, wherein said variant is the one identified in SEQ ID NO: 1.

37. A transgenic non-human animal containing a gene construct according to any of claims 19 to 21 inserted in its genome.


SEQUENCE LISTING

<110>  UNIVERSIDAD AUTÓNOMA DE MADRID

<120>  METHOD FOR THE IDENTIFICATION OF COMPOUNDS FOR THE TREATMENT OF
       NEURODEGENERATIVE DISEASES

<130>  P3185EPPC

<150>  ES P200702997
<151>  2007-11-13

<160>  8

<170>  PatentIn version 3.5

<210>  1
<211>  1686
<212>  DNA
<213>  Homo sapiens

<400>  1
ccggccttgt gacagtttct acttaaactc tggctgcccc catgagggta tgacaggata      60

tgtaacaggc caccacatgg cctctgtttt caaggcacaa gaaaaatact ttgcattctc     120

ggtggtgcgt tttacttttg gtcaagttgc ctccaagttc atggttctca ttttaccttc     180

acaactttta tctttaattt acaaacaagg acggtaagat cccttggcaa gtgatgggga     240

gttgagtcta aaatcgcggc tccggtctcc ccagtccagg gcagtcctgc gctgaagtgc     300

actcacacgt cccgggatga gactccgctg taaatctcga cccgaacacc taggaggcag     360

gcctgtgccg cgctcctcac acagggagtc acaggacttg ctgtctggta cagcaagaga     420

gggtcttgga gaaagccata ggagtaccgt gggggaaaat aggtggccaa acagaatcgg     480

gtccactggg caaaatacat gacttctggc aggctgaggg cttaccaact acaaggtaaa     540

agtcaagggc gctgttctga gagaagcgga aaatgactac caagggattc cagtacctcg     600

gccccttttg ggagatttac ggggctagaa caggagacca cccccgtttt tttgtttgtt     660

tgttttgttt tttgtttttg gtgaaacgta gtctcgctct gtcacccagg ctggagtgca     720

gtggcgcgat ctcggtcact gcaacatccg cctccagggt tcaagcgatt cttctgcctc     780

tgcctccaga gtagctggga ttacaggcgc gcatcaccac acccggctaa tttttgtatt     840

tttagtagag acggggtttc accatgttgg tcaggctgat ctcgaactcc tgacctcaag     900

tgatccgctc tcctcagcct cccaaagtgc tgggattaca ggcgtaagcc actgagcccg     960

gccaggagac ctctttaaga agactcgaga tgtcgacaat cccagtggat ggataccaac    1020

tttaaaaaga aaagttcaaa aggcctatgt gcccattagc tgggaggggc caagaaatat 1080

gggagtccct tatagtgggg gtaaaacggc gggtaaagct aggtgggcgg aacagcagct 1140

tctgggggag acaagcggca aagaggctca cgaccgacta ggggcgacca aggctgatag 1200

ccgttggggc cgttgggcgc cgggagctgg cgccccgccc tctctcctct cccccaccct 1260

ccgcacctcc caccaccctc ggtgtcccct gcgcgtgcgc gtgcagacac cggttgccaa 1320

acattgcatc atccccgccc ccctttcctc ccctcccccc ccgctacact cccctccgcg 1380

cgcgcgcatg actcacccac ctcctccgtg aagcctcgtg acccaaagcc acttccgggt 1440

ccgacactac gtcgaccccc tagcgagagg gagcgacggg ggcggtgccg cggggctcct 1500

gagtggcgga tgcgagggac ggggcggggc caatgccggc gtgccacttt ctgattggta 1560

ggttttgggg tcccgcccct gagaggaggg caaggccatg gtaaaagatt acagccaggc 1620

gctcccgagg tcagagactt aagtctaagg cactgagcgt atcatgttaa agatgagcgg 1680

gtggca 1686

<210> 2
<211> 1686
<212> DNA
<213> Homo sapiens

<400> 2
ccggccttgt gacagtttct acttaaactc tggctgcccc catgagggta tgacaggata 60

tgtaacaggc caccacatgg cctctgtttt caaggcacaa gaaaaatact ttgcattctg 120

ggtggtgcgt tttacttttg gtcaagttgc ctccaagttc atggttctca ttttaccttc 180

acaactttta tctttaattt acaaacaagg acggtaagat cccttggcaa gtgatgggga 240

gttgagtcta aaatcgcggc tccggtctcc ccagtccagg gcagtcctgc gctgaagtgc 300

actcacacgt cccgggatga gactccgctg taaatctcga cccgaacacc taggaggcag 360

gcctgtgccg cgctcctcac acagggagtc acaggacttg ctgtctggta cagcaagaga 420

gggtcttgga gaaagccata ggagtaccgt gggggaaaat aggtggccaa acagaatcgg 480

gtccactggg caaaatacat gacttctggc aggctgaggg cttaccaact acaaggtaaa 540

agtcaagggc gctgttctga gagaagcgga aaatgactac caagggattc cagtacctcg 600

gccccttttg ggagatttac ggggctagaa caggagacca cccccgtttt tttgtttgtt 660

tgttttgttt tttgtttttg gtgaaacgta gtctcgctct gtcacccagg ctggagtgca 720

gtggcgcgat ctcggtcact gcaacatccg cctccagggt tcaagcgatt cttctgcctc 780

tgcctccaga gtagctggga ttacaggcgc gcatcaccac acccggctaa tttttgtatt 840

tttagtagag acggggtttc accatgttgg tcaggctgat ctcgaactcc tgacctcaag 900

tgatccgctc tcctcagcct cccaaagtgc tgggattaca ggcgtaagcc actgagcccg 960

gccaggagac ctctttaaga agactcgaga tgtcgacaat cccagtggat ggataccaac 1020

tttaaaaaga aaagttcaaa aggcctatgt gcccattagc tgggaggggc caagaaatat 1080

gggagtccct tatagtgggg gtaaaacggc gggtaaagct aggtgggcgg aacagcagct 1140

tctgggggag acaagcggca aagaggctca cgaccgacta ggggcgacca aggctgatag 1200

ccgttggggc cgttgggcgc cgggagctgg cgccccgccc tctctcctct cccccaccct 1260

ccgcacctcc caccaccctc ggtgtcccct gcgcgtgcgc gtgcagacac cggttgccaa 1320

acattgcatc atccccgccc ccctttcctc ccctcccccc ccgctacact cccctccgcg 1380

cgcgcgcatg actcacccac ctcctccgtg aagcctcgtg acccaaagcc acttccgggt 1440

ccgacactac gtcgaccccc tagcgagagg gagcgacggg ggcggtgccg cggggctcct 1500

gagtggcgga tgcgagggac ggggcggggc caatgccggc gtgccacttt ctgattggta 1560

ggttttgggg tcccgcccct gagaggaggg caaggccatg gtaaaagatt acagccaggc 1620

gctcccgagg tcagagactt aagtctaagg cactgagcgt atcatgttaa agatgagcgg 1680

gtggca 1686

<210> 3
<211> 34
<212> DNA
<213> Artificial

<220>
<223> Oligonucleotide Chop-PromU

<400> 3
ctagtagctt ggatccggcc ttgtgacagt ttct 34

<210> 4
<211> 33
<212> DNA
<213> Artificial

<220>
<223> Oligonucleotide Chop-PromL

<400>  4
tatacactac aagcttgcca cccgctcatc ttt                                    33


<210>  5
<211>  26
<212>  DNA
<213>  Artificial

<220>
<223>  Oligonucleotide Ddit3-MutU

<400>  5
actttgcatt ctgggtggtg cgtttt                                            26


<210>  6
<211>  26
<212>  DNA
<213>  Artificial

<220>
<223>  Oligonucleotide Ddit3-MutL

<400>  6
aaaacgcacc acccagaatg caaagt                                            26


<210>  7
<211>  24
<212>  DNA
<213>  Artificial

<220>
<223>  Oligonucleotide 1

<400>  7
ccttgattga caaggatgga tggc                                              24


<210>  8
<211>  26
<212>  DNA
<213>  Artificial

<220>
<223>  Oligonucleotide 2

<400>  8
catcgtcggg aagacctgcc acgccc                                            26